# EUROPEAN PATENT APPLICATION

(11) **EP 1 932 562 A1**
(43) Date of publication of application: **18.06.2008**
(21) Application number: 06797792.6
(22) Date of filing: 11.09.2006
(51) Int. Cl.: A61N 1/30, A61N 1/04

(54) **IONTOPHORESIS APPARATUS**

(30) Priority: 14.09.2005 JP 2005266623
(71) Applicant: Transcu Ltd., 048580 Singapore (SG)
(72) Inventor: AKIYAMA, Hidero c/o TTI ellebeau Inc., Shinagawa-ku, Tokyo, 140-0002 (JP); KANAMURA, Kiyoshi c/o TTI ellebeau Inc., Shinagawa-ku, Tokyo, 140-0002 (JP); NAKAYAMA, Mizuo c/o TTI ellebeau Inc., Shinagawa-ku, Tokyo, 140-0002 (JP); MATSUMURA, Takehiko c/o TTI ellebeau Inc., Shinagawa-ku, Tokyo, 140-0002 (JP); MATSUMURA, Akihiko c/o TTI ellebeau Inc., Shinagawa-ku, Tokyo, 140-0002 (JP)
(74) Representative: Schäfer, Matthias W.
(86) International application number: PCT/JP2006/317972
(87) International publication number: WO 2007/032307

(57) **Abstract**

Energization from an electrode to a drug holding portion is performed through an ionic liquid. An iontophoresis device capable of preventing the generation of a gas or an unpreferable ion upon energization, or an iontophoresis device capable of preventing the alteration of a drug ion due to an electrode reaction is realized by selecting an anion constituting the ionic liquid from PF₆⁻, BF₄⁻, AlCl₄⁻, ClO₄⁻, a hydrogensulfate ion, bis-trifluoroalkylsulfonyl-imide, and trifluoromethanesulfonate, and selecting a cation constituting the ionic liquid from an imidazolium derivative, a pyridinium derivative, a piperidinium derivative, a pyrolidinium derivative, and a tetraalkylammonium derivative.

## Description

### FIELD OF THE INVENTION

The present invention relates to an iontophoresis device. In particular, the present invention relates to an iontophoresis device capable of preventing or suppressing an unpreferable electrode reaction in an electrode assembly.

### BACKGROUND OF THE INVENTION

Iontophoresis involves driving a drug dissociated to plus or minus ions in a solution by means of a voltage to transdermally transfer the drug into an organism, and has advantages such as a reduced load to a subject and excellent controllability of the amount of the drug to be administered.

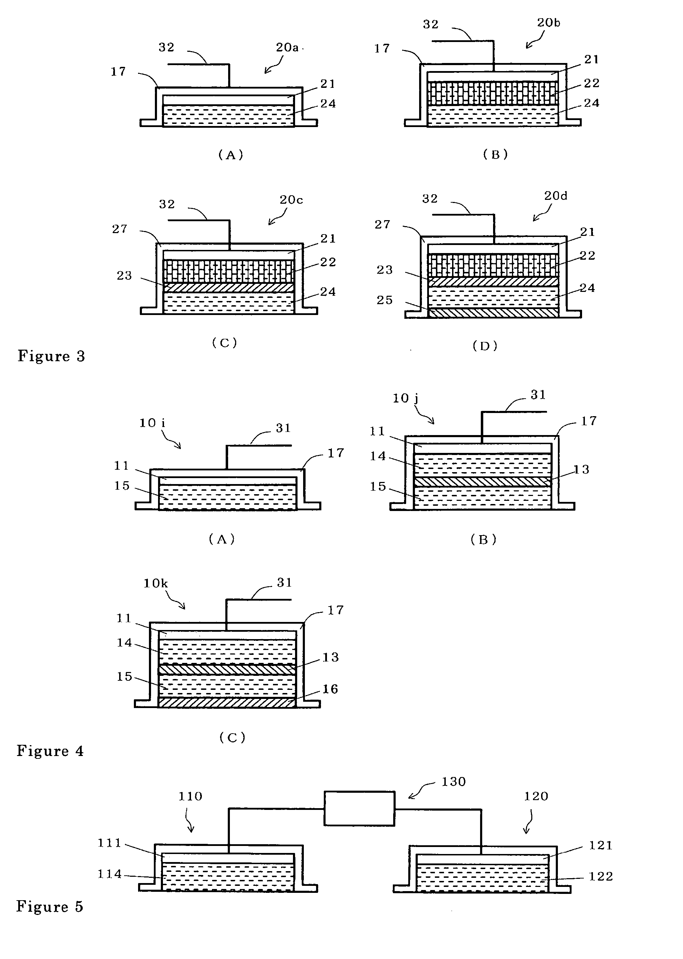

Fig. 5 is an explanatory view showing the basic constitution of an iontophoresis device as a device for performing the above-mentioned iontophoresis.

As shown in the figure, the iontophoresis device includes: a working electrode assembly 110 having an electrode 111 and a drug solution holding portion 114 holding a solution of a drug that dissociates to plus or minus drug ions (a drug solution); a non-working electrode assembly 120 having an electrode 121 and an electrolyte solution holding portion 122 holding an electrolyte solution; and an electric power source 130 with both of its terminals connected to the electrodes 111 and 121. A voltage having the same conductivity type as that of a drug ion is applied to the electrode 111 and a voltage having a conductivity type opposite to that of the drug ion is applied to the electrode 121 in a state where the drug solution holding portion 114 and the electrolyte solution holding portion 122 are brought into contact with the skin of an organism. As a result, the drug ion is administered to the organism.

One of the problems to be solved in such iontophoresis device is various electrode reactions occurring in the electrode assemblies 110 and 120.

For example, in the case where a drug is a cationic drug that dissociates to plus drug ions, a hydrogen ion or an oxygen gas may be generated at the electrode 111 and a hydroxide ion or a hydrogen gas may be generated at the electrode 121 by the electrolysis of water. In addition, a drug ion may alter owing to an electrode reaction depending on the kind of the drug. Furthermore, when the drug solution holding portion 114 contains a chlorine ion, a chlorine gas or hypochlorous acid may be generated.

Similarly, in the case where a drug is an anionic drug that dissociates to minus drug ions, a hydroxide ion or a hydrogen gas may be generated at the electrode 111 and a hydrogen ion or an oxygen gas may be generated at the electrode 121 by the electrolysis of water. In addition, a drug ion may alter owing to an electrode reaction depending on the kind of the drug. Furthermore, when the electrolyte solution holding portion 122 contains a chlorine ion, a chlorine gas or hypochlorous acid may be generated.

When such gas as described above is generated in the electrode assembly 110 or 120, energization from the electrode 111 or 121 to the drug solution or the electrolyte solution is inhibited. When a hydrogen ion, a hydroxide ion, and hypochlorous acid are generated in the electrode assembly 110 or 120, they transfer to a biological interface to have a detrimental effect on an organism. In addition, the alteration of a drug may cause unpreferable conditions such as the inability to obtain an initial drug effect and the production of a toxic substance.

Patent Document 1 discloses, as an iontophoresis device capable of solving such problems as described above, an iontophoresis device in which a silver electrode is used as an anode and a silver chloride electrode is used as a cathode.

In the iontophoresis device, a reaction preferentially occurs, in which silver in the anode is oxidized by energization to become insoluble silver chloride, while silver chloride is reduced at the cathode to become metal silver. As a result, the generation of various gases and the production of various ions due to such electrode reactions as described above can be suppressed.

However, it is difficult to prevent the dissolution of the silver electrode during storage of the iontophoresis device. In particular, in the case where the device is intended for administering a cationic drug, the number of kinds of applicable drugs is extremely limited. In addition, a morphological change upon production of silver chloride from the silver electrode is large. Therefore, special consideration must be given in order to prevent such morphological change from affecting the properties of the device. As a result, there arises a problem in that a severe restriction is imposed on the shape of the device (for example, a lamination structure cannot be adopted). Furthermore, the iontophoresis device is not always able to solve the problem of the alteration of a drug upon energization.

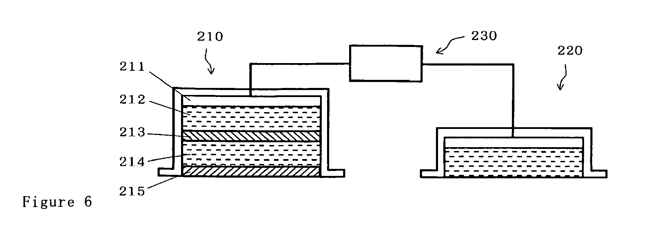

Patent Document 2 discloses, as another iontophoresis device capable of solving the above problems, an iontophoresis device shown in Fig. 6.

As shown in the figure, the iontophoresis device is constituted by: a working electrode assembly 210 including an electrode 211, an electrolyte solution holding portion 212 holding an electrolyte solution in contact with the electrode 211, an ion exchange membrane 213 of a second conductivity type, the ion exchange membrane 213 being placed on the front surface side of the electrolyte solution holding portion 212, a drug solution holding portion 214 holding a drug solution containing a drug ion of a first conductivity type, the drug solution holding portion 214 being placed on the front surface side of the ion exchange membrane 213, and an ion exchange membrane 215 of the first conductivity type, the ion exchange membrane 215 being placed on the front surface side of the drug solution holding portion 214; and a non-working electrode assembly 220 and an electrode 230 similar to those shown in Fig. 9.

In the iontophoresis device, the electrolyte solution and the drug solution are partitioned by the second ion exchange membrane 213 of the second conductivity type. As a result, the composition of the electrolyte solution can be selected independently of the drug solution. Accordingly, an electrolyte solution containing no chlorine ion can be used, and the selection of an electrolyte having a lower oxidation or reduction potential than the electrolysis of water as the electrolyte in the electrolyte solution can suppress the production of an oxygen gas, a hydrogen gas, a hydrogen ion, or a hydroxide ion resulting from the electrolysis of water. Furthermore, in the iontophoresis device, the transfer of a drug ion to the electrolyte solution holding portion is blocked by the second ion exchange membrane, so a problem in that the drug ion alters owing to an electrode reaction is solved.

However, the iontophoresis device described in Patent Document 2 involves a problem in that it is difficult to completely separate the drug solution of the drug holding portion 214 from the electrolyte solution of the electrolyte solution holding portion 212.

That is, ion of the first and second conductivity types generated as a result of the ion dissociation of an electrolyte and undissociated electrolyte molecules are generally coexistent in the electrolyte solution of the electrolyte solution holding portion 212. However, an ion of the second conductivity type and an electrolyte molecule can pass through the ion exchange membrane 213 to transfer to the drug solution holding portion 214. Therefore, depending on kinds of a drug ion and the electrolyte or a combination of them, during the storage of the device over a certain time period or longer, those ions or molecules may transfer to the drug holding portion 214 to act on the drug ion, thereby providing an unpreferable influence such as a reduction in drug effect or a change in color.
[Patent Document 1] US 4,744,787
[Patent Document 2] JP 4-297277 A

### DISCLOSURE OF INVENTION

### PROBLEMS TO BE SOLVED

An object of the present invention is to provide an iontophoresis device which is capable of preventing or suppressing the generation of an oxygen gas, a chlorine gas, or a hydrogen gas in an electrode assembly as in the case of each of Patent Documents 1 and 2, and which has a constitution different from that of each of Patent Documents 1 and 2.

Another object of the present invention is to provide an iontophoresis device which is capable of preventing or suppressing the generation of a hydrogen ion, a hydroxide ion, or hypochlorous acid in an electrode assembly as in the case of each of Patent Documents 1 and 2, and which has a constitution different from that of each of Patent Documents 1 and 2.

Another object of the present invention is to provide an iontophoresis device which is capable of preventing or suppressing the alteration of a drug ion due to an electrode reaction upon energization as in the case of Patent Document 2, and which has a constitution different from that of Patent Document 2.

Another object of the present invention is to provide an iontophoresis device which is capable of preventing or suppressing the generation of a gas or an ion or the alteration of a drug as described above, and which causes no large change in morphology of an electrode due to energization.

Another object of the present invention is to provide an iontophoresis device which is capable of preventing or suppressing the generation of a gas or an ion or the alteration of a drug as described above, which is capable of preventing the alteration of a drug ion due to an electrode reaction upon energization as in the case of Patent Document 2, and which has a constitution different from that of Patent Document 2.

Another object of the present invention is to provide an iontophoresis device which is capable of: preventing or suppressing the generation of a gas or an ion or the alteration of a drug as described above; and reducing the possibility that the alteration of a drug or a change in color during the storage of the device occurs lower than that of the iontophoresis device described in Patent Document 2.

### MEANS FOR SOLVING PROBLEMS

The present invention relates to an iontophoresis device
characterized by including a working electrode assembly including: a first electrode supplied with a voltage of a first conductivity type; an ionic liquid holding portion holding ionic liquid in contact with the first electrode; and a drug holding portion holding a drug ion of the first conductivity type, placed on a front surface side of the ionic liquid holding portion (claim 1).

In the present invention, energization from the first electrode to the ionic liquid is caused by the oxidation or reduction of a cation or anion constituting the ionic liquid. Accordingly, energization in the working electrode assembly can be performed without the generation of a gas such as an oxygen gas, a chlorine gas, or a hydrogen gas, or the generation of a harmful ion such as a hydrogen ion, a hydroxyl ion, or hypochlorous acid.

The term "ionic liquid" as used herein refers to a molten salt present as a liquid at around normal temperature.

An anion constituting the ionic liquid of the present invention can be selected from PF₆⁻, BF₄⁻, AlCl₄⁻, Clo₄⁻, a hydrogensulfate ion represented by the following formula (1), bis-trifluoroalkylsulfonyl-imide represented by the following formula (2), trifluoromethanesulfonate represented by the following formula (3), and a combination of them. 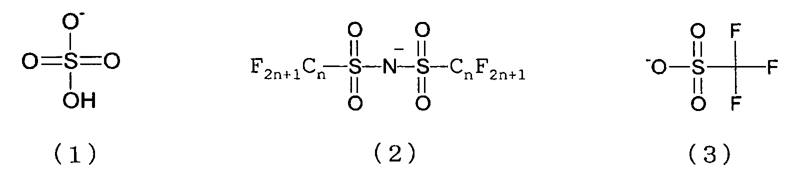 It should be noted that n in the formula (2) represents an arbitrary natural number.

A cation constituting the ionic liquid of the present invention can be selected from: an imidazolium derivative containing monoalkylimidazolium represented by the following formula (4), dialkylimidazolium represented by the following formula (5), or trialkylimidazolium represented by the following formula (6); a pyridinium derivative containing 1-alkylpyridinium represented by the following formula (7); a piperidinium derivative containing dialkylpiperidinium represented by the following formula (8); a pyrolidinium derivative containing 1-alkylpyrolidinium represented by the following formula (9); a tetraalkylammonium derivative containing tetraalkylammonium represented by the following formula (10); and a combination of them. 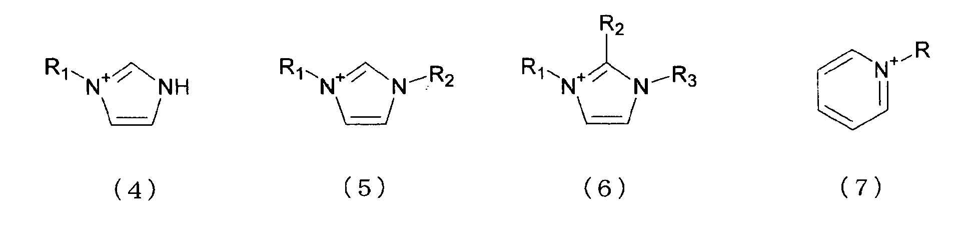 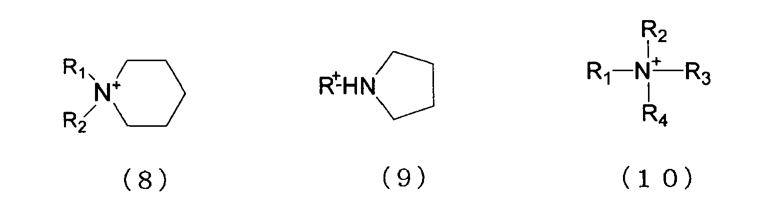 It should be noted that R and R1 to R4 in the formulae (4) to (10) each represent an arbitrary alkyl or fluoroalkyl group.

A hydrophobic liquid is preferably used for the ionic liquid in the present invention (claim 2). In this case, in addition to the above-described basic effect of the present invention, an additional effect, that is, an ability to improve separability between the ionic liquid holding portion and the drug holding portion can be achieved.
Accordingly, it becomes possible to prevent: the occurrence of, for example, the alteration of a drug ion or a change in color of a drug solution due to the transfer of an anion or cation constituting the ionic liquid to the drug holding portion; and a reduction in safety to a human body.
Alternatively, it becomes possible to prevent the alteration of a drug ion due to an electrode reaction as a result of the transfer of the ion to the ionic liquid holding portion.

Such hydrophobic ionic liquid can be constituted by selecting bis-trifluoroalkylsulfonyl-imide described above as an anion and selecting any one of the cations exemplified above as a cation.

The ionic liquid in the present invention is preferably blended with an electrolyte having a lower oxidation-reduction potential that that of the ionic liquid (claim 5).

That is, the oxidation-reduction potential of an ionic liquid is about 3 to 5 V in many cases. Therefore, when it is desirable to administer a drug at a reduced applied voltage, dissolving an electrolyte having a lower oxidation-reduction potential than that of the ionic liquid can reduce a voltage necessary to be applied to the first electrode for the administration of a drug.

In this case, an electrolyte that does not generate a gas or a harmful ion owing to oxidation-reduction is preferably selected. In some cases, an electrolyte that does not cause a phenomenon such as the alteration of a drug ion or a change in color of a drug solution even when the electrolyte or an ion generated as a result of the dissociation of the electrolyte transfers to the drug holding portion is preferably selected.

The invention according to claim 5 has an advantage in that the range of choices of a sacrificial electrolyte (an electrolyte which has a lower oxidation-reduction potential than that of the ionic liquid or water as a solvent, or which does not generate a gas or a harmful ion owing to oxidation-reduction, or an electrolyte which dose not cause a phenomenon such as the alteration of a drug ion upon transfer to the drug holding portion) can be extended as compared to the iontophoresis device described in Patent Document 2 because the oxidation-reduction potential of the ionic liquid is much higher than the oxidation-reduction potential (1.23 V) of water as described above.

Examples of the sacrificial electrolyte with which the ionic liquid can be blended include: inorganic compounds such as ferrous sulfate and ferric sulfate; drugs such as ascorbic acid and sodium ascorbate; acidic compounds such as lactic acid each present on the surface of a skin; and organic acids such as oxalic acid, malic acid, succinic acid, and fumaric acid and/or salts of them.

In the inventions according to any one of claims 1 to 5, it is preferable that: the drug holding portion hold a drug solution containing the drug ion and a drug counter ion of a second conductivity type; and the working electrode assembly further include a semi-permeable membrane allowing passage of at least the drug counter ion between the drug holding portion and the ionic liquid holding portion (claim 6), thereby further ensuring the separation of the ionic liquid holding portion from the drug holding portion.

An ion exchange membrane of the second conductivity type can be used as the semi-permeable membrane here. In this case, while energization from the ionic liquid holding portion to the drug holding portion is secured by the transfer of the drug counter ion to the ionic liquid holding portion, the transfer of an ion of the first conductivity type present in the ionic liquid holding portion to the drug holding portion and the transfer of the drug ion to the ionic liquid holding portion can be blocked by means of the ion exchange membrane of the second conductivity type. Accordingly, it becomes possible to prevent: the alteration of a drug solution due to the transfer of the ion of the first conductivity type in the ionic liquid holding portion to the drug holding portion; or a reduction in safety to an organism. Alternatively, it becomes possible to prevent the alteration of a drug ion due to an electrode reaction that has transferred to the ionic liquid holding portion.

A membrane filter having molecular weight cutoff property with which the passage of the drug ion and/or the ion of the first conductivity type in the ionic liquid holding portion can be blocked while the passage of the drug counter ion is permitted can also be used as the semi-permeable membrane here. In this case as well, an effect similar to that in the case where the ion exchange membrane of the second conductivity type is used can be achieved.

In the invention according to any one of claims 1 to 6, the working electrode assembly preferably further includes an ion exchange membrane of the first conductivity type on the front surface side of the drug holding portion (claim 7). In this case, the efficiency of the administration of the drug ion can be increased because the ion exchange membrane of the first conductivity type blocks the transfer of a biological counter ion to the drug holding portion upon administration of a drug.

In the inventions according to any one of claims 1 to 5, it is preferable that: the drug holding portion be composed of an ion exchange membrane of the first conductivity type doped with the drug ion; the working electrode assembly further include an electrolyte solution holding portion holding an electrolyte solution, placed on a front surface side of a semi-permeable membrane, and the semi-permeable membrane allowing passage of at least an ion of a second conductivity type of the electrolyte solution holding portion, placed between the ionic liquid holding portion and the drug holding portion; and the drug holding portion be placed on a front surface side of the electrolyte solution holding portion (claim 8), thereby further ensuring the separation of the ionic liquid holding portion from the electrolyte solution holding portion and/or the drug holding portion.

An ion exchange membrane or membrane filter similar to that described above with respect to the invention according to claim 6 can be used as the semi-permeable membrane here, and an effect similar to that of the invention according to claim 6 can be achieved.

In addition, the invention according to claim 8 achieves the following additional effects together.
(1) The efficiency of the administration of a drug can be increased because the ion exchange membrane of the first conductivity type blocks the transfer of a biological counter ion to the device.
(2) The efficiency of the administration of a drug ion can be additionally increased because the administration of the drug ion is performed in a state where an ion exchange membrane holding the drug ion is brought into direct contact with the skin of an organism.
(3) The stability of a drug ion during storage increases, and a reduction in amount of a stabilizer, an antibacterial agent, an antiseptic, or the like to be used or the extension of a time period for which the device is stored can be attained because the drug ion is held in a state where the first ion exchange membrane is doped with the ion.

In the inventions according to any one of claims 1 to 8, it is preferable that the iontophoresis further include a non-working electrode assembly having: a second electrode supplied with a voltage of the second conductivity type; a second ionic liquid holding portion holding ionic liquid in contact with the second electrode; and a second electrolyte solution holding portion holding an electrolyte solution, placed on a front surface side of the second ionic liquid holding portion (claim 9).

The invention can solve a problem in that a gas or a harmful ion generates owing to energization even in the non-working electrode assembly because energization from the second electrode to the ionic liquid is caused by the oxidation or reduction of a cation or anion constituting the ionic liquid.

The second ionic liquid holding portion can hold an ionic liquid similar to that described above with respect to the ionic liquid holding portion in a manner similar to that described above with respect to the ionic liquid holding portion.

An iontophoresis device of the present invention can include a working electrode assembly having a first electrode and a drug holding portion holding a drug ion of a first conductivity type, the drug ion being administered to an organism by a voltage of a first conductivity type applied to the first electrode, the iontophoresis device being
characterized by further including a non-working electrode assembly having: a second electrode supplied with a voltage of a second conductivity type; a second ionic liquid holding portion holding ionic liquid in contact with the second electrode; and a second electrolyte solution holding portion holding an electrolyte solution, placed on a front surface side of the second ionic liquid holding portion (claim 10).

Energization from the second electrode to the ionic liquid in the present invention is caused by the oxidation or reduction of a cation or anion constituting the ionic liquid. Accordingly, energization in the non-working electrode assembly can be performed without the generation of a gas such as an oxygen gas, a nitrogen gas, or a hydrogen gas, or the generation of a harmful ion such as a hydrogen ion, a hydroxyl ion, or hypochlorous acid.

The second ionic liquid holding portion in the present invention can hold an ionic liquid similar to that described above with respect to the ionic liquid holding portion in a manner similar to that described above with respect to the ionic liquid holding portion.

The working electrode assembly in the present invention can be constituted in the same manner as in the working electrode assembly 110 shown in Fig. 5. When the working electrode assembly in the present invention is constituted in the same manner as in Patent Document 1 or 2, the generation of a gas or a harmful ion can be suppressed. Alternatively, when the working electrode assembly in the present invention is constituted in the same manner as in the working electrode assembly in the invention according to claim 1, the generation of a gas or a harmful ion can be suppressed.

The term "drug" as used herein refers to a substance which may be or may not be prepared, which has a certain drug effect or pharmacological action, and which is applicable to an organism for purposes including the therapy, recovery, or prevention of a disease, the promotion or maintenance of the health, the diagnosis of a disease condition, a health condition or the like, or the promotion or maintenance of the beauty.

The term "drug ion" as used herein refers to an ion which is produced by the dissociation of a drug to ions and which plays a role in a drug effect or a pharmacological action. The dissociation of the drug to a drug ion may occur as a result of the dissolution of the drug into a solvent such as water, an alcohol, an acid, or an alkali, or may occur as a result of, for example, the application of a voltage or the addition of an ionizing agent.

The term "drug solution" as used herein refers to a liquid-like solution prepared by dissolving a drug into a solvent. In addition, the term includes various states such as one prepared by suspending or emulsifying a drug into a solvent and one adjusted to be an ointment state or a paste state as long as at least part of the drug dissociates to drug ions in the solvent.

The term "drug counter ion" as used herein refers to an ion present in a drug solution and having a conductivity type opposite to that of a drug ion.

The term "skin" as used herein refers to the surface of an organism to which a drug can be administered by iontophoresis, and includes, for example, a mucosa in an oral cavity. The term "organism" as used herein refers to a human being or an animal.

The term "biological counter ion" as used herein refers to an ion present on the skin of an organism or in the organism and having a conductivity type opposite to that of a drug ion.

The term "front surface side" as used herein means that a positional relationship on a current path upon administration of a drug is close to a skin. The term "β arranged on the front surface side of α" means that β is positioned so as to be closer to a skin than α upon administration of a drug.

The term "first conductivity type" as used herein refers to plus or minus electrical polarity, and the term "second conductivity type" as used herein refers to the electrical polarity (minus or plus) opposite to the first conductivity type.

Each of a cation and an anion in the ionic liquid holding portion in the present invention is not necessarily of a single kind, and one or both of them may be of multiple kinds. In the same manner, each of a drug ion and a drug counter ion in the drug holding portion, or each of a plus ion and a minus ion in the electrolyte solution of the electrolyte solution holding portion is not necessarily of a single kind, and one or both of them may be of multiple kinds.

Known examples of an ion exchange membrane include various ion exchange membranes such as a heterogenenous ion exchange membrane obtained by: dispersing an ion exchange resin in a binder polymer; and forming the resultant into a membrane through, for example, molding under heat and a homogeneous ion exchange membrane obtained by: impregnating a base material such as cloth, a net, or a porous film with a solution prepared by dissolving a composition composed of a monomer, a cross-linkable monomer, a polymerization initiator, or the like into which an ion exchange group can be introduced or a resin having a functional group into which an ion exchange group can be introduced into a solvent; subjecting the resultant to polymerization or solvent removal; and subjecting the resultant to a treatment for introducing an ion exchange group, in addition to an ion exchange membrane obtained by forming an ion exchange resin into a membrane shape.

More specifically, an ion exchange membrane into which a cation exchange group is introduced such as a NEOSEPTA (CM-1, CM-2, CMX, CMS, or CMB) manufactured by Tokuyama Co., Ltd can be used for the cation exchange membrane. An ion exchange membrane into which an anion exchange group is introduced such as a NEOSEPTA (AM-1, AM-3, AMX, AHA, ACH, or ACS) manufactured by Tokuyama Co. , Ltd can be used for the anion exchange membrane.

The term "ion exchange membrane of a first conductivity type" as used herein refers to an ion exchange membrane having a function of selectively passing an ion of the first conductivity type, that is, an ion exchange membrane through which an ion of the first conductivity type passes more easily than an ion of the second conductivity type. When the first conductivity type is plus, the "ion exchange membrane of the first conductivity type" is a cation exchange membrane. When the first conductivity type is minus, the "ion exchange membrane of the first conductivity type" is an anion exchange membrane.

Similarly, the term "ion exchange membrane of a second conductivity type" as used herein refers to an ion exchange membrane having a function of selectively passing an ion of the second conductivity type, that is, an ion exchange membrane through which an ion of the second conductivity type passes more easily than an ion of the first conductivity type. When the second conductivity type is plus, the "ion exchange membrane of the second conductivity type" is a cation exchange membrane. When the second conductivity type is minus, the "ion exchange membrane of the second conductivity type" is an anion exchange membrane.

Examples of a cation exchange group to be introduced to the cation exchange membrane include a sulfonic group, a carboxylic group, and a phosphoric group. The transport number of an ion exchange membrane can be controlled depending on the kind of a cation exchange group to be introduced. For example, the use of a sulfonic group as a strong acidic group can provide a cation exchange membrane having a high transport number.

Examples of an anion exchange group to be introduced to the anion exchange membrane include a primary amino group, a secondary amino group, a tertiary amino group, a quaternary ammonium group, a pyridyl group, an imidazole group, a quaternary pyridinium group, and a quaternary imidazolium group. The transport number of an ion exchange membrane can be controlled depending on the kind of an anion exchange group to be introduced. For example, the use of a quaternary ammonium group or a quaternary pyridinium group as a strong basic group can provide an anion exchange membrane having a high transport number.

Known examples of a treatment for introducing a cation exchange group include various approaches such as sulfonation, chlorosulfonation, phosphonation, and hydrolysis. Known examples of a treatment for introducing an anion exchange group include various approaches such as amination and alkylation. The transport number of an ion exchange membrane can be adjusted by adjusting conditions under which a treatment for introducing an ion exchange group is performed.

In addition, the transport number of an ion exchange membrane can be adjusted depending on, for example, the amount of an ion exchange resin in the ion exchange membrane and the pore size of the membrane. For example, in the case of an ion exchange membrane of a type in which a porous film is filled with an ion exchange resin, an ion exchange membrane obtained by filling a porous film with an ion exchange resin at a filling ratio of preferably 5 to 95 mass%, more preferably 10 to 90 mass%, or particularly preferably 20 to 60 mass% can be used, the porous film having formed thereon a large number of small pores having a mean pore size of preferably 0.005 to 5.0 µm, more preferably 0.01 to 2.0 µm, or most preferably 0.02 to 0.2µm (a mean flow pore size measured in conformance with the bubble point method (JIS K3832-1990)) at a porosity of preferably 20 to 95%, more preferably 30 to 90%, or most preferably 30 to 60% and having a thickness of preferably 5 to 140 µm, more preferably 10 to 120 µm, or most preferably 15 to 55 µm. The transport number of the ion exchange membrane can also be adjusted depending on the mean pore size of the small pores of the porous film, the porosity, and the filling ratio of the ion exchange resin.

The term "blocking of the passage of an ion" to be described for an ion exchange membrane in the specification does not always mean the blocking of the passage of all ions. The term includes the case where, even the passage of an ion occurs with a certain speed, the degree of the passage is so small that the passage of a drug ion is suppressed to the extent that no alteration of a drug occurs near an electrode even if the device is stored over a practically sufficient period or the passage of a biological counter ion is suppressed to the extent that the efficiency of administration of the drug can be sufficiently increased.

Similarly, the term "permission of the passage of an ion" to be described for an ion exchange membrane in the specification does not mean that no restrictions are imposed on the passage of an ion. The term includes the case where an ion is allowed to pass with a sufficiently high speed or amount as compared to an ion having a conductivity type opposite to that of the former ion even when the passage of the ion is restricted to some extent.

The terms "blocking of the passage of an ion or a molecule " and "permission of the passage of an ion or a molecule" to be described for a semi-permeable membrane in the specification have the same meanings as those described above, and do not mean the blocking of the passage of all ions or molecules or that no restrictions are imposed on the passage of an ion or a molecule.

### BREIF DESCRIPTION OF THE DRAWINGS

[Fig. 1] An explanatory view showing the schematic constitution of an iontophoresis device according to an embodiment of the present invention.
[Figs. 2] Figs. 2 (A) to 2 (H) are explanatory sectional views each showing the constitution of a working electrode assembly of an iontophoresis device according to an embodiment of the present invention.
[Figs. 3] Figs. 3(A) to 3(D) are explanatory sectional views each showing the constitution of a non-working electrode assembly of an iontophoresis device according to an embodiment of the present invention.
[Figs. 4] Figs. 4 (A) to 4 (C) are explanatory sectional views each showing the constitution of a working electrode assembly of an iontophoresis device according to an embodiment of the present invention.
[Fig. 5] An explanatory view showing the constitution of a conventional iontophoresis device.
[Fig. 6] An explanatory view showing the constitution of another conventional iontophoresis device.

### BEST MODE FOR CARRYING OUT THE INVENTION

Hereinafter, an embodiment of the present invention will be described with reference to the drawings.

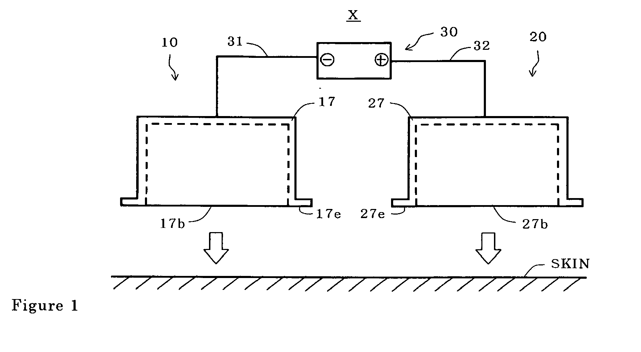

Fig. 1 is an explanatory view showing the schematic constitution of an iontophoresis device X according to the present invention.

In the following, for convenience of description, an iontophoresis device for administering a drug whose drug component dissociates to plus drug ions (for example, lidocaine hydrochloride or morphine hydrochloride) will be exemplified. An iontophoresis device for administering a drug whose drug component dissociates to minus ions (for example, ascorbic acid), the device being capable of achieving substantially the same effect as that of the following embodiment, can be constituted by reversing the pole of an electric power source, the conductivity type of each of each ion exchange membrane and an ion with which a doping layer or a cation exchange membrane is doped in the following description.

As shown in the figure, the iontophoresis device X includes: an electric power source 30; a working electrode assembly 10 connected to the plus pole of the electric power source 30 with an electric supply line 31; and a non-working electrode assembly 20 connected to the minus pole of the electric power source 30 with an electric supply line 32.

The working electrode assembly 10 and the non-working electrode assembly 20 each include a container 17 or 27 having formed therein a space capable of housing various structures to be described later.

The container 17 or 27 can be formed of an arbitrary material such as a plastic, but is preferably formed of a flexible material capable of: preventing the evaporation of water from the inside of the container and the penetration of foreign matter from the outside; and following the movement of an organism or the irregularities of the skin of the organism. In addition, a lower surface 17b or 27b of the container 17 or 27 is opened, a removable liner composed of an appropriate material for preventing the evaporation of water and the mixing of foreign matter during storage of the iontophoresis device X can be stuck to the lower surface 17b or 27b of the container 17 or 27. An adhesive layer for improving adhesiveness to a skin upon administration of a drug can be arranged on a lower end portion 17e or 27e of the container 17 or 27.

A battery, a constant voltage device, a constant current device, a constant voltage/current device, or the like can be used as the electric power source 30. It is preferable to use a constant current device whose current can be adjusted in the range of 0.01 to 1.0 mA/cm², or preferably 0.01 to 0.5 mA/cm², and which operates under safe voltage conditions at 50 V or less, or preferably 30 V or less.

The iontophoresis device X administers a drug ion to an organism through energization from the electric power source 30 in a state where the lower surfaces 17b and 27b of the working electrode assembly 10 and the non-working electrode assembly 20 are brought into contact with the skin of an organism.

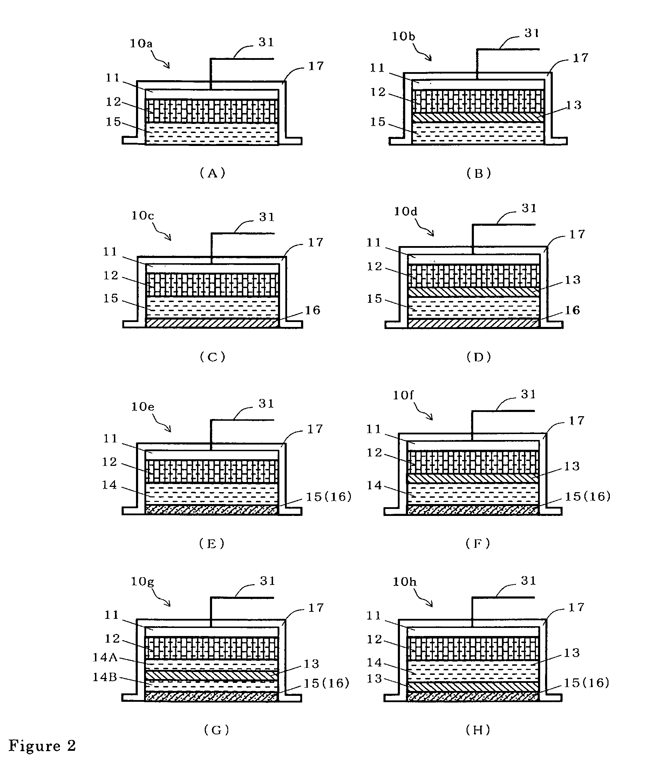

Figs. 2(A) to 2(H) are explanatory sectional views showing the constitutions of working electrode assemblies 10a to 10h each of which can be used as the working electrode assembly 10 of the iontophoresis device X.

The working electrode assembly 10a of Fig. 2(A) includes: an electrode 11 connected to the electric supply line 31 of the electric power source 30; an ionic liquid holding portion 12 holding an ionic liquid in contact with the electrode 11; and a drug holding portion 15 holding a drug solution, the drug holding portion 15 being arranged on the front surface side of the ionic liquid holding portion 12.

An electrode composed of an arbitrary conductive material can be used for the electrode 11 without any particular limitation. It is preferable to use an inactive electrode composed of gold, platinum, carbon, or the like rather than an active electrode composed of silver or the like in order to avoid a change in morphology of the electrode 11.

The ionic liquid of the ionic liquid holding portion 12 is a salt molten at normal temperature constituted by: an anion selected from PF₆⁻, BF₄⁻, AlCl₄⁻, ClO₄⁻, a hydrogensulfate ion, bis-trifluoroalkylsulfonyl-imide, trifluoromethanesulfonate, and a combination of them; and a cation selected from an imidazolium derivative, a pyridinium derivative, a piperidinium derivative, a pyrolidinium derivative, a tetraalkylammonium derivative, and a combination of them.

When bis-trifluoroalkylsulfonyl-imide is selected as the anion of the ionic liquid, hydrophobicity can be imparted to the ionic liquid. Therefore, separability between the ionic liquid of the ionic liquid holding portion 12 and the drug solution of the drug holding portion 15 can be improved.

In addition, the above ionic liquid can be blended with an electrolyte having a lower oxidation potential than that of the ionic liquid. The blending can reduce a voltage necessary for causing energization from the electrode 11 to the ionic liquid holding portion 12.

Examples of an electrolyte that can be used for such purpose include: ferrous sulfate; ferric sulfate; ascorbic acid; sodium ascorbate; and lactic acid, oxalic acid, malic acid, succinic acid, and fumaric acid, or salts of them.

The ionic liquid holding portion 12 can hold the ionic liquid in a liquid state. Alternatively, the portion can hold the ionic liquid in a state where an appropriate absorbing carrier (such as a microporous body or a sponge-like polymer (for example, a polyimide porous membrane or a polytetrafluoroethylene microporous membrane)) is impregnated with the ionic liquid. In this case, separability between the ionic liquid of the ionic liquid holding portion 12 and the drug solution of the drug holding portion 15 can be improved.

The drug solution of the drug holding portion 15 is a solution of a drug whose drug component dissociates to plus drug ions. The drug holding portion 15 can hold the drug solution in a liquid state. Alternatively, when the portion holds the drug solution with which an appropriate absorbing carrier such as a gauze, filter paper, or a gel is impregnated, separability between the ionic liquid of the ionic liquid holding portion 12 and the drug solution of the drug holding portion 15 can be improved.

In the working electrode assembly 10a, a drug ion in the drug holding portion 15 is administered to an organism by applying a plus voltage to the electrode 11 in a state where the drug holding portion 15 is brought into contact with the skin of an organism. Energization from the electrode 11 to the ionic liquid holding portion 12 in this case is caused by the oxidation of an anion or cation constituting the ionic liquid. Alternatively, when the ionic liquid is blended with an electrolyte having a lower oxidation potential than that of the ionic liquid, energization from the electrode 11 to the ionic liquid holding portion 12 is caused by the oxidation of the electrolyte. Accordingly, the generation of an oxygen gas or a chlorine gas, and the production of a hydrogen ion or hypochlorous acid due to energization that have been of concern in a conventional iontophoresis device are suppressed.

Energization from the ionic liquid holding portion 12 to the drug holding portion 15 is mainly caused by the transfer of a drug counter ion in the drug holding portion 15 to the ionic liquid holding portion 12.

Furthermore, a cation constituting the ionic liquid seldom transfers to the drug holding portion 15 owing to energization from the ionic liquid holding portion 12 to the drug holding portion 15 because any one of the above-described cations each constituting the ionic liquid is hydrophobic. Accordingly, a problem such as the alteration of a drug ion or a reduction in safety to an organism due to the transfer of the cation constituting the ionic liquid to the drug holding portion 15 can be avoided.

In an iontophoresis device administering a drug whose drug component dissociates to minus drug ions, when the transfer of an anion constituting the ionic liquid to the drug holding portion 15 upon energization must be prevented, selecting bis-trifluoroalkylsulfonyl-imide as the anion constituting the ionic liquid can prevent the transfer.

The working electrode assembly 10b of Fig. 2(B) includes: the electrode 11, the ionic liquid holding portion 12, and the drug holding portion 15 similar to those of the working electrode assembly 10a; and the anion exchange membrane 13 between the ionic liquid holding portion 12 and the drug holding portion 15.

The working electrode assembly 10b achieves an effect similar to that described above with respect to the working electrode assembly 10a, and achieves the following additional effects because the anion exchange membrane 13 blocks the transfer of a drug ion to the ionic liquid holding portion 12 and the transfer of plus ions in the ionic liquid holding portion 12 (a cation constituting the ionic liquid and a plus ion generated by the dissociation of an electrolyte with which the ionic liquid is blended) to the drug holding portion 15.
(1) The alteration of the drug ion due to an electrode reaction is prevented.
(2) The alteration of the drug ion or a reduction in safety to an organism due to the plus ions that have transferred from the ionic liquid holding portion 12 to the drug holding portion 15 is prevented.

In order that the above effects may be achieved with improved sufficiency, an anion exchange membrane having as high a transport number as possible is preferably used for the anion exchange membrane 13. An anion exchange membrane prepared by filling the pores of a porous film with an anion exchange resin is particularly preferably used therefor.

The working electrode assembly 10c of Fig. 2(C) includes: the electrode 11, the ionic liquid holding portion 12, and the drug holding portion 15 similar to those of the working electrode assembly 10a; and a cation exchange membrane 16 on the front surface side of the drug holding portion 15.

The working electrode assembly 10c achieves an effect similar to that described above with respect to the working electrode assembly 10a, and achieves an additional effect, that is, an increase in transport number of a drug ion upon administration of a drug because the cation exchange membrane 16 blocks the transfer of a biological counter ion from the side of an organism to the drug holding portion 15.

The working electrode assembly 10d of Fig. 2(D) includes: the electrode 11, the ionic liquid holding portion 12, the anion exchange membrane 13, and the drug holding portion 15 similar to those of the working electrode assembly 10b; and a cation exchange membrane 16 on the front surface side of the drug holding portion 15.

The working electrode assembly 10d achieves an effect similar to that described above with respect to the working electrode assembly 10b, and achieves an additional effect, that is, an increase in transport number of a drug ion upon administration of a drug because the cation exchange membrane 16 blocks the transfer of a biological counter ion from the side of an organism to the drug holding portion 15.

In each of the working electrode assemblies 10c and 10d, a cation exchange membrane having as high a transport number as possible is preferably used for the cation exchange membrane 16 for improving an increasing effect on the transport number of a drug ion. A cation exchange membrane prepared by filling the pores of a porous film with a cation exchange resin is particularly preferably used therefor.

The anion exchange membrane 13 in each of the working electrode assemblies 10b and 10d can be replaced with a membrane filter capable of blocking the passage of a drug ion and/or a plus ion in the ionic liquid holding portion 12 while permitting the passage of a drug counter ion. In this case as well, an effect similar to that of each of the working electrode assemblies 10b and 10d can be achieved.

The working electrode assembly 10e of Fig. 2(E) includes: the electrode 11 and the ionic liquid holding portion 12 similar to those of the working electrode assembly 10a; an electrolyte solution holding portion 14 holding an electrolyte solution, the electrolyte solution holding portion 14 being arranged on the front surface side of the ionic liquid holding portion 12; and the drug holding portion 15 composed of the cation exchange membrane 16 doped with a drug ion, the drug holding portion 15 being arranged on the front surface side of the electrolyte solution holding portion 14.

The electrolyte solution holding portion 14 can hold an arbitrary electrolyte solution for securing conductivity from the ionic liquid holding portion 12 to the drug holding portion 15. However, an electrolyte solution free of any plus ion having a mobility comparable to or lower than that of a drug ion is preferably used in order that the transport number of the drug ion upon energization may be additionally increased.

The electrolyte solution holding portion 14 can hold the electrolyte solution in a liquid state. Alternatively, when the portion holds the electrolyte solution with which an appropriate absorbing carrier such as a gauze, filter paper, or a gel is impregnated, separability between the ionic liquid of the ionic liquid holding portion 12 and the electrolyte solution of the electrolyte solution holding portion 14 can be improved.

One similar to the cation exchange membrane of each of the working electrode assemblies 10c and 10d can be used for the cation exchange membrane 16. The cation exchange membrane 16 can be doped with a drug ion by immersing the cation exchange membrane 16 in a drug solution having an appropriate concentration. The amount of a drug ion with which the cation exchange membrane 16 is doped can be adjusted depending on, for example, the concentration of a drug solution to be used at this time, an immersion time period, and the number of times of immersion. When the cation exchange membrane 16 is doped with a drug ion, the drug ion is considered to bind to a cation exchange group in the cation exchange membrane 16 through an ionic bond.

Energization from the electrode 11 to the ionic liquid holding portion 12 in the working electrode assembly 10e occurs in a manner similar to that of the working electrode assembly 10a. Therefore, the generation of an oxygen gas or a chloride gas, and the production of a hydrogen ion or hypochlorous acid due to energization can be suppressed.

Energization from the ionic liquid holding portion 12 to the electrolyte solution holding portion 14 is mainly caused by the transfer of a minus ion in the electrolyte solution holding portion 14 to the ionic liquid holding portion 12. Energization from the electrolyte solution holding portion 14 to the drug holding portion 15 is caused by the transfer of a plus ion in the electrolyte solution holding portion 14 to the drug holding portion 15 . A drug ion with which the cation exchange membrane 16 of the drug holding portion 15 is doped is substituted by a plus ion from the electrolyte solution holding portion 14, to be thereby administered to an organism.

The working electrode assembly 10e achieves the following additional effects as well as an effect similar to that described above with respect to the working electrode assembly 10a.
(1) The efficiency of the administration of a drug ion is increased because the cation exchange membrane 16 blocks the transfer of a biological counter ion to the drug holding portion 15.
(2) The efficiency of the administration of the drug ion is additionally increased because the administration of the drug ion is performed in a state where the cation exchange membrane 16 doped with the drug ion is brought into direct contact with the skin of an organism.
(3) The stability of a drug ion during storage increases, and a reduction in amount of a stabilizer, an antibacterial agent, an antiseptic, or the like to be used or the extension of a time period for which the device is stored can be attained because the drug ion is held in a state where the cation exchange membrane 16 is doped with the ion.

The working electrode assembly 10f of Fig. 2(F) includes: the electrode 11, the ionic liquid holding portion 12, the electrolyte solution holding portion 14, and the drug holding portion 15 similar to those of the working electrode assembly 10e; and the anion exchange membrane 13 between the ionic liquid holding portion 12 and the electrolyte solution holding portion 14.

An anion exchange membrane similar to that described above with respect to the working electrode assembly 10b can be used for the anion exchange membrane 13.

The working electrode assembly 10f achieves an effect similar to that described above with respect to the working electrode assembly 10e, and achieves the following additional effects because the movement of a plus ion between the ionic liquid holding portion 12 and the electrolyte solution holding portion 14 is blocked.
(1) The alteration of a drug ion in the cation exchange membrane 16 due to an electrode reaction upon energization is prevented because the transfer of the drug ion to the ionic liquid holding portion 12 via the electrolyte solution holding portion 14 is prevented.
(2) The alteration of a drug ion or a reduction in safety to an organism is prevented because the transfer of a plus ion in the ionic liquid holding portion 12 to the drug holding portion 15 via the electrolyte solution holding portion 14 is prevented.

The anion exchange membrane 13 in the working electrode assembly 10f can be replaced with a membrane filter capable of blocking the passage of a plus ion in the ionic liquid holding portion 12 (especially a cation constituting the ionic liquid) while permitting the passage of a minus ion in the electrolyte solution holding portion 14. In this case as well, an effect similar to that of the working electrode assembly 10f can be achieved.

The working electrode assembly 10g of Fig. 2(G) is different from the working electrode assembly 10f only in that: two electrolyte solution holding portions 14A and 14B are arranged between the ionic liquid holding portion 12 and the drug holding portion 15; and the anion exchange membrane 13 is arranged between the two electrolyte solution holding portions 14A and 14B.

The working electrode assembly 10g achieves an effect similar to that of to the working electrode assembly 10f via a mechanism similar to that described above with respect to the working electrode assembly 10f.

The working electrode assembly 10h of Fig. 2(H) includes: the electrode 11, the ionic liquid holding portion 12, the electrolyte solution holding portion 14, and the drug holding portion 15 similar to those of the working electrode assembly 10e; and the anion exchange membrane 13 between the electrolyte solution holding portion 14 and the drug holding portion 15.

An anion exchange membrane having a slight low transport number (for example, a transport number of 0.7 to 0.98) is used for the anion exchange membrane 13 in the working electrode assembly 10h.

Energization from the electrode 11 to the ionic liquid holding portion 12 and energization from the ionic liquid holding portion 12 to the electrolyte solution holding portion 14 in the working electrode assembly 10h each occur in a manner similar to that described above with respect to the working electrode assembly 10e.

Energization from the electrolyte solution holding portion 14 to the drug holding portion 15 is caused by the transfer of a plus ion in the electrolyte solution holding portion 14, which has passed through the anion exchange membrane 13, to the drug holding portion 15. A drug ion with which the cation exchange membrane 16 of the drug holding portion 15 is doped is substituted by a plus ion from the electrolyte solution holding portion 14, to be thereby transferred to an organism.

Accordingly, the working electrode assembly 10h achieves an effect similar to that of the working electrode assembly 10f.

Figs. 3 (A) to 3 (D) are explanatory sectional views showing the constitutions of non-working electrode assemblies 20a to 20d each of which can be used as the non-working electrode assembly 20 of the above-described iontophoresis device X.

The non-working electrode assembly 20a of Fig. 3(A) includes:
the electrode 21 connected to an electric supply line 32; an electrolyte solution holding portion 24 holding an electrolyte solution in contact with the electrode 21.

The use of an active electrode composed of silver chloride or the like for the electrode 21 can prevent the generation of a hydrogen gas or a hydroxyl ion due to the electrolysis of water. As described later, an inactive conductive electrode composed of gold, platinum, carbon, or the like can also be used when an electrolyte solution prepared by dissolving an electrolyte having a lower reduction potential than that of water is used as the electrolyte solution of the electrolyte solution holding portion 24.

The electrolyte solution holding portion 24 can hold an arbitrary electrolyte solution for securing energization property from the electrode 21 to an organism. When an electrolyte solution prepared by dissolving an electrolyte having a lower reduction potential than that of water or a buffer electrolyte solution prepared by dissolving multiple kinds of electrolytes is used, the generation of a hydrogen gas due to an electrode reaction and a fluctuation in pH due to the production of a hydrogen ion can be prevented.

Examples of the electrolyte which can be preferably used as an electrolyte in the electrolyte solution can include: inorganic compounds such as ferrous sulfate and ferric sulfate; drugs such as ascorbic acid and sodium ascorbate; acidic compounds each present on the surface of a skin such as lactic acid; and organic acids such as oxalic acid, malic acid, succinic acid, and fumaric acid and/or salts of them.

The electrolyte solution holding portion 24 can hold the electrolyte solution in a liquid state. Alternatively, when the portion holds the electrolyte solution with which an appropriate absorbing carrier such as a gauze, filter paper, or a gel is impregnated, the handleability or the like of the electrolyte solution can be improved.

The non-working electrode assembly 20a can serve as a counter electrode of the working electrode assembly 10. The non-working electrode assembly 20a closes a current path ranging from the plus pole of the electric power source 30 to the minus pole of the electric power source 30 via the working electrode assembly 10, an organism, and the non-working electrode assembly 20a.

The non-working electrode assembly 20b of Fig. 3(B) includes: the electrode 21 connected to an electric supply line 32; an ionic liquid holding portion 22 holding an ionic liquid in contact with the electrode 21; and the electrolyte solution holding portion 24 arranged on the front surface side of the ionic liquid holding portion 22.

An electrode composed of an arbitrary conductive material can be used for the electrode 21 of the non-working electrode assembly 20b without any particular limitation. It is preferable to use an inactive electrode composed of gold, platinum, carbon, or the like rather than an active electrode composed of silver chloride or the like in order to avoid a change in morphology of the electrode 21.

The ionic liquid holding portion 22 can be constituted in a manner similar to that of the ionic liquid holding portion 12.

The electrolyte solution holding portion 24 holds an electrolyte solution for securing energization property from the ionic liquid holding portion 12 to an organism, and can hold an arbitrary electrolyte solution such as a saline having high safety to the organism.

The electrolyte solution holding portion 24 can hold the electrolyte solution in a liquid state. Alternatively, when the portion holds the electrolyte solution with which an appropriate absorbing carrier such as a gauze, filter paper, or a gel is impregnated, separability between the ionic liquid of the ionic liquid holding portion 22 and the electrolyte solution of the electrolyte solution holding portion 24 can be improved.

In the non-working electrode assembly 20b, energization from the electrode 21 to the ionic liquid holding portion 22 is caused by the reduction of an anion or a cation constituting the ionic liquid. Alternatively, when the ionic liquid is blended with an electrolyte having a lower reduction potential than that of the ionic liquid, the energization is caused by the reduction of the electrolyte.

Accordingly, the non-working electrode assembly 20b plays a role similar to that of the non-working electrode assembly 20a, and achieves an additional effect, that is, the suppression of the production of a hydrogen gas or a hydroxyl ion that has been of concern in a conventional iontophoresis device.

The non-working electrode assembly 20c of Fig. 3(C) includes: the electrode 21, the ionic liquid holding portion 22 and the electrolyte solution holding portion 24 similar to those of the non-working electrode assembly 20b; and the cation exchange membrane 23 being placed between the ionic liquid holding portion 22 and the electrolyte solution holding portion 24.

The non-working electrode assembly 20c achieves an effect similar to that described above with respect to the non-working electrode assembly 20b. In addition, the cation exchange membrane 23 blocks the transfer of a minus ion from the ionic liquid holding portion 22 to the electrolyte solution holding portion 24. Accordingly, a reduction in safety to an organism that may occur upon transfer of a minus ion in the ionic liquid holding portion 22 to the electrolyte solution holding portion 24 can be prevented.

In order that this effect may be achieved with improved sufficiency, a cation exchange membrane having as high a transport number as possible is preferably used for the cation exchange membrane 23. A cation exchange membrane prepared by filling the pores of a porous film with a cation exchange resin is particularly preferably used therefor.

The non-working electrode assembly 20d of Fig. 3(D) includes: the electrode 21, the ionic liquid holding portion 22, the cation exchange membrane 23 and the electrolyte solution holding portion 24 similar to those of the non-working electrode assembly 20c; and the anion exchange membrane 25 being placed on the front surface side of the electrolyte solution holding portion 24.

The non-working electrode assembly 20d achieves an effect similar to that described above with respect to the non-working electrode assembly 20c, and achieves an additional effect, that is, an ability to maintain an ion balance at an interface between the anion exchange membrane 25 and a skin with improved favorableness because the anion exchange membrane 25 is arranged on the front surface side of the electrolyte solution holding portion 24.

Figs. 4(A) to 4(C) are explanatory sectional views showing the constitutions of working electrode assemblies 101 to 10k each of which can be used as the working electrode assembly 10 of the above-described iontophoresis device X and each of which can be combined with the non-working electrode assembly 20b, 20c, or 20d to constitute the iontophoresis device X according to the present invention.

The working electrode assembly 101 of Fig. 4(A) includes: the electrode 11 connected to the electric supply line 31 of the electric power source 30; and the drug holding portion 15 holding a drug solution in contact with the electrode 11.

The drug holding portion 15 of the working electrode assembly 10i can be constituted in a manner similar to that of the drug holding portion 15 of the working electrode assembly 10a. A silver electrode can be used for the electrode 11 for preventing the generation of an oxygen gas or a chlorine gas due to an electrode reaction, or the production of a hydrogen ion.

The iontophoresis device X including the working electrode assembly 10i achieves the following effect. That is, the generation of a gas or the production of a harmful ion in each of the non-working electrode assemblies 20b to 20d is suppressed by an ionic liquid while a drug ion is administered from the working electrode assembly 10i to an organism.

The working electrode assembly 10j of Fig. 4(B) includes: the electrode 11 connected to the electric supply line 31 of the electric power source 30; the electrolyte solution holding portion 14 holding an electrolyte solution in contact with the electrode 11; the anion exchange membrane 13 arranged on the front surface side of the electrolyte solution holding portion 14; and the drug holding portion 15 holding a drug solution, the drug holding portion 15 being arranged on the front surface side of the anion exchange membrane 13.

The drug holding portion 15 in the working electrode assembly 10j can be constituted in a manner similar to that of the drug holding portion of the working electrode assembly 10a. An anion exchange membrane similar to that described above with respect to the working electrode assembly 10b can be used for the anion exchange membrane 13 of the working electrode assembly 10j.

An electrolyte solution prepared by dissolving an electrolyte having a lower oxidation potential than that of water or a buffer electrolyte solution prepared by dissolving multiple kinds of electrolytes can be used as the electrolyte solution of the electrolyte solution holding portion 14 in the working electrode assembly 10j. In this case, the generation of a hydrogen gas or a hydrogen ion due to an electrode reaction can be prevented even when an inactive electrode composed of gold, platinum, carbon, or the like is used for the electrode 11.

The working electrode assembly 10j achieves an effect similar to that described above with respect to the working electrode assembly 10i, and achieves an additional effect, that is, an ability to prevent the alteration of a drug ion due to an electrode reaction upon energization because the anion exchange membrane 13 blocks the transfer of the drug ion from the drug holding portion 15 to the electrolyte solution holding portion 14.

The working electrode assembly 10k of Fig. 4(C) includes: the electrode 11, the electrolyte solution holding portion 14, the anion exchange membrane 13, and the drug holding portion 15 similar to those of the working electrode assembly 10j; and the cation exchange membrane 16 arranged on the front surface side of the drug holding portion 15.

A cation exchange membrane similar to that described above with respect to the working electrode assembly 10c can be used for the cation exchange membrane 16.

The working electrode assembly 10k achieves an effect similar to that of the working electrode assembly 10j, and achieves an additional effect, that is, an increase in transport number of a drug ion because the cation exchange membrane 16 blocks the transfer of a biological counter ion from the side of an organism to the drug holding portion 15.

The present invention has been described above by way of several embodiments. However, the present invention is not limited to those embodiments, and can be variously altered within the scope of claims.

For example, in each of the above embodiments, description has been given of the case where a single working electrode assembly and a single non-working electrode assembly are connected to each of both poles of an electric power source. However, the case where multiple working electrode assemblies or multiple non-working electrode assemblies are used, or the case where multiple working electrode assemblies and multiple non-working electrode assemblies are used is also permitted. In this case, the scope of the present invention includes an iontophoresis device in which at least one of the multiple working electrode assemblies is the working electrode assembly specified in claim 1, or an iontophoresis device in which at least one of the multiple non-working electrode assemblies is the non-working electrode assembly specified in claim 11.

In each of the above embodiments, description has been made by taking the case where an iontophoresis device includes a non-working electrode assembly as an example. However, the iontophoresis device itself can be provided with no non-working electrode assembly. That is, a drug can be administered by: bringing a working electrode assembly into contact with the skin of an organism; and applying a voltage to the working electrode assembly in a state where part of the organism is brought into contact with a member to serve as an earth. In this case aswell, the basic effect of the present invention, that is, the suppression of the generation of an oxygen gas, a hydrogen gas, a chlorine gas, or the like or the suppression of the production of a hydrogen ion, a hydroxyl ion, or hypochlorous acid in the working electrode assembly upon energization is achieved, and such iontophoresis device is also included in the scope of the present invention.

In each of the above embodiments, description has been given of the case where a drug ion is administered from only a working electrode assembly connected to one pole of an electric power source. Alternatively, one or multiple working electrode assemblies can be connected to each of both poles of the electric power source so that positive and negative drug ions are simultaneously administered from the one or multiple working electrode assemblies connected to each of both the poles of the electric power source. In this case, the scope of the present invention includes an iontophoresis device in which at least one of those working electrode assemblies is the working electrode assembly specified in claim 1.

Furthermore, in each of the above embodiments, description has been given of the case where the working electrode assembly, the non-working electrode assembly, and the electric power source are constituted separately. It is also possible that those elements are incorporated in a single casing or an entire device incorporating them is formed in a sheet shape or a patch shape, with the result that the handleability thereof is enhanced, and such iontophoresis device is also included in the scope of the present invention.

## Claims

1. An iontophoresis device **characterized by** comprising a working electrode assembly comprising:
a first electrode supplied with a voltage of a first conductivity type;
an ionic liquid holding portion holding ionic liquid in contact with the first electrode; and
a drug holding portion holding a drug ion of the first conductivity type, placed on a front surface side of the ionic liquid holding portion.

2. The iontophoresis device according to claim 1, **characterized in that** the ionic liquid is hydrophobic.

3. The iontophoresis device according to claim 1 or 2, **characterized in that** an anion constituting the ionic liquid is bis-trifluoroalkyl-sulphonyl-imide.

4. The iontophoresis device according to any one of claims 1 to 3, **characterized in that** a cation constituting the ionic liquid is an imidazorium derivative, a pyridinium derivative, a piperidinium derivative, a pyrrolidinium derivative, or a tetraalkylammonium derivative.

5. The iontophoresis device according to any one of claims 1 to 4, **characterized in that** an electrolyte having an oxidation-reduction potential lower than an oxidation-reduction potential of the ionic liquid is dissolved in the ionic liquid.

6. The iontophoresis device according to any one of claims 1 to 5, **characterized in that**:
the drug holding portion holds a drug solution containing the drug ion and a drug counter ion of a second conductivity type; and
the working electrode assembly further comprises a semi-permeable membrane allowing passage of at least the drug counter ion between the drug holding portion and the ionic liquid holding portion.

7. The iontophoresis device according to any one of claims 1 to 6, **characterized in that** the working electrode assembly further includes an ion exchange membrane of the first conductivity type on a front surface side of the drug holding portion.

8. The iontophoresis device according to any one of claims 1 to 5, **characterized in that**:
the drug holding portion is composed of an ion exchange membrane of the first conductivity type doped with the drug ion;
the working electrode assembly further includes an electrolyte solution holding portion holding an electrolyte solution, placed on a front surface side of the ionic liquid holding portion, and a semi-permeable membrane allowing passage of at least an ion of a second conductivity type of the electrolyte solution holding portion, placed between the ionic liquid holding portion and the drug holding portion; and
the drug holding portion is placed on a front surface side of the electrolyte solution holding portion.

9. The iontophoresis device according to any one of claims 1 to 8, **characterized by** further comprising a non-working electrode assembly having:
a second electrode supplied with a voltage of the second conductivity type;
a second ionic liquid holding portion holding ionic liquid in contact with the second electrode; and
a second electrolyte solution holding portion holding an electrolyte solution, placed on a front surface side of the second ionic liquid holding portion.

10. An iontophoresis device comprising a working electrode assembly having a first electrode and a drug holding portion holding a drug ion of a first conductivity type, the drug ion being administered to a living body by a voltage of a first conductivity type applied to the first electrode, the iontophoresis device being **characterized by** further comprising a non-working electrode assembly having:
a second electrode supplied with a voltage of a second conductivity type;
a second ionic liquid holding portion holding ionic liquid in contact with the second electrode; and
an electrolyte solution holding portion holding an electrolyte solution, placed on a front surface side of the second ionic liquid holding portion.
